(19) Europäisches Patentamt
European Patent Office
Office européen des brevets

(11) Veröffentlichungsnummer: **0 069 066**
**B1**

(12) # EUROPÄISCHE PATENTSCHRIFT

(45) Veröffentlichungstag der Patentschrift: **03.10.84**

(21) Anmeldenummer: **82810255.8**

(22) Anmeldetag: **14.06.82**

(51) Int. Cl.³: **C 07 C 69/732**, C 07 C 69/738, C 07 C 67/347, C 07 C 39/11, C 07 C 69/145, C 07 C 91/32, C 07 D 257/06, C 07 C 103/78, C 07 C 143/78, C 07 F 9/145 // C07C91/06

(54) **Resorcinole und deren Herstellung.**

(30) Priorität: **19.06.81 GB 8119011**

(43) Veröffentlichungstag der Anmeldung: **05.01.83 Patentblatt 83/1**

(45) Bekanntmachung des Hinweises auf die Patenterteilung: **03.10.84 Patentblatt 84/40**

(84) Benannte Vertragsstaaten: **BE DE FR GB IT**

(56) Entgegenhaltungen: **Keine Entgegenhaltungen.**

(73) Patentinhaber: **CIBA-GEIGY AG, Postfach, CH-4002 Basel (CH)**

(72) Erfinder: **Howell, Frederick Harold, Dr., 27 High Bank, Atherton Lancashire M29 9HZ (GB)**

EP 0 069 066 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Resorcinole und ein Verfahren zu deren Herstellung.

Resorcinole mit direkt an den aromatischen Ring gebundenen funktionellen Gruppen sind bekannt und z.B. in „Rodd's Chemistry of Carbon Compounds", 2. Aufl. Bd. III D und in der US Patentschrift Nr. 4126461 beschrieben. Seit langem ist bekannt, dass gewisse phenolische Verbindungen, besonders Resorcinole, als photographische Farbkuppler zum Erzeugen Bilder neutraler Dichte oder schwarzer Bilder verwendet werden können. Bisher bestand jedoch kein sehr grosses Bedürfnis, schwarze Bilder herzustellen, so dass solche phenolische Farbkuppler kaum zur Anwendung gelangten. Wegen des hohen Silberpreises ist nun aber ein sehr grosses Bedürfnis entstanden, das Silber in photographischen Bild-Materialien auf Silberhalogenid-Basis zu ersetzen, oder das Silberbild durch Verwendung schwarzer Farbstoffe zu verstärken. Die bekannten phenolischen Verbindungen ergeben jedoch keine schwarzen Bilder von ausreichend guter Farbe oder Dichte.

Die US Patentschrift Nr. 4126461 beschreibt die Verwendung bestimmter Resorcinole als schwarze Farbkuppler. Diese Resorcinole ergeben bei der Farbentwicklung annähernd befriedigende schwarze Bilder. Sie sind jedoch sehr schwer zugänglich und das Abtrennen von nichtkuppelnden Nebenprodukten aus diesen Resorcinolen hat sich als sehr schwierig erwiesen.

In der britischen Patentschrift Nr. 1564349 und in der offengelegten britischen Patentanmeldung Nr. 2044474 sind weitere phenolische Verbindungen als schwarze Farbkuppler beschrieben. Die m-Aminophenolverbindungen gemäss der britischen Patentschrift Nr. 1564349 ergeben beim Farbkuppeln in Farbentwicklungsverfahren dunkelblaue Farben [Farbtöne], die unterhalb 500 nm eine geringe Dichte aufweisen, weshalb sie sowohl als End-Bildfarbstoffe als auch als Negativ-Bildfarbstoffe bei der Herstellung von Positivbildern praktisch keine Verwendung fanden. Die britische Offenlegungsschrift Nr. 2044474 beschreibt Verbindungen, bei denen zwei Pyrazolonkerne über ein 4-substituiertes Phenol miteinander verbunden sind. Diese Verbindungen ergeben beim Farbkuppeln in Farbentwicklungsverfahren visuell neutrale schwarze Bilder. Das Spektrum ihrer Farbabsorption ist jedoch sehr unregelmässig und weist mehrere Spitzen auf, so dass sie nicht als Negativbilder zum Herstellen von Positivbildern verwendet werden können.

Es wurden nun neue, durch tertiäre Alkylgruppen mit funktionellen Gruppen substituierte Resorcinole gefunden, mit denen die obigen Nachteile überwunden werden können und die beim Kuppeln in photographischen Farbentwicklungsverfahren gute, tief schwarze Farbbilder ergeben, die allein oder zusammen mit Silberbildern als Endfarbbild in Positivbildern oder aber für Negativbilder zur Herstellung von Positivbildern verwendet werden können.

Die neuen Resorcinole entsprechen der Formel I

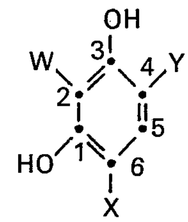

(I)

worin

W Wasserstoff, Halogen, n-Alkyl mit 1 bis 5 C-Atomen, $-NHCOR^1$, $-COR^1$ oder eine Phenoxyacetylaminogruppe, die durch eine oder zwei $C_{1-12}$-Alkylgruppen substituiert sein kann,

X eine Gruppe in Kupplungsposition und zwar Wasserstoff, Chlor, Brom, $-SR^{11}$ oder den Rest eines über ein Ring-N-Atom gebundenen N-Heterocyclus,

Y eine Gruppe der Formel II

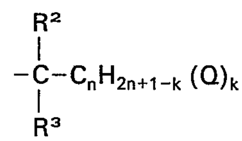

(II) und

Q $-COOR^4$, $-CONR^4R^5$, $-OR^5$, $-OCOR^6$, $-NR^7R^8$, $-PO(OR^9)[O]_xR^{10}$ mit x=0 oder 1, $-SO_2OH$, $-SO_2NR^4R^5$ oder $-CN$ bedeuten,

n eine ganze Zahl von 1 bis 20 ist,

k 1 oder 2 darstellt,

$R^1$ $C_{1-12}$-Alkyl, $C_{2-12}$-Alkenyl, $C_{3-8}$-Cycloalkyl, $C_{7-13}$-Aralkyl oder $C_{6-10}$-Aryl bedeutet, das durch eine oder zwei $C_{1-4}$-Alkylgruppen substituiert sein kann,

$R^2$ und $R^3$ unabhängig voneinander $C_{1-5}$-Alkyl darstellen, wobei bei $Q=-COOR^4$ $R^2$ oder $R^3$ durch eine oder zwei $-COOR^4$-Gruppen substituiert sein kann, oder mindestens eines von $R^2$ und $R^3$ so an den Rest $-C_nH_{2n+1-k}$ gebunden ist, dass ein durch $-(COOR^4)_k$ substituierter $C_{5-12}$-Cycloalkylenrest entsteht, wobei die Gruppen $R^4$ gleiche oder verschiedene Bedeutungen haben können,

$R^4$ Wasserstoff, $C_{1-20}$-Alkyl, das durch ein oder mehrere O-Atome unterbrochen sein kann, $C_{3-20}$-Alkenyl, $C_{3-12}$-Cycloalkyl, $C_{7-13}$-Aralkyl oder gegebenenfalls substituiertes $C_{6-10}$-Aryl, und

$R^5$ Wasserstoff oder $C_{1-20}$-Alkyl bedeuten, oder

$R^4$ und $R^5$ zusammen mit dem N-Atom, an das sie gebunden sind, einen 5- oder 6gliedrigen heterocyclischen Ring bilden, der durch eine $C_{1-4}$-Alkylgruppe substituiert sein kann, bevorzugt einen Pyrrolidin-, Piperidin- oder Morpholinring,

$R^6$ Wasserstoff, $C_{1-20}$-Alkyl oder $C_{3-20}$-Alkenyl, $C_{3-12}$-Cycloalkyl, $C_{7-13}$-Aralkyl oder gegebenenfalls durch eine oder zwei $C_{1-4}$-Alkylgruppen substituiertes $C_{6-10}$-Aryl,

$R^7$ Wasserstoff oder $C_{1-4}$-Alkyl, und

$R^8$ Wasserstoff, $C_{1-4}$-Alkyl oder $-COR^4$ darstellen, oder

$R^7$ und $R^8$ zusammen mit dem N-Atom, an das sie gebunden sind, einen 5- oder 6gliedrigen heterocyclischen Ring bilden, der durch eine $C_{1-4}$-Alkylgruppe substituiert sein kann, bevorzugt einen Pyrrolidin-, Piperidin-, Morpholin- oder 3,5-Dimethylmorpholinring,

$R^9$ Wasserstoff oder $C_{1-20}$-Alkyl,

$R^{10}$ bei x=1 Wasserstoff oder $C_{1-20}$-Alkyl und bei x=0 $C_{1-5}$-Alkyl bedeuten oder $R^9$ und $R^{10}$ zusammen $C_{2-3}$-Alkylen bilden, das durch 1 oder mehrere $C_{1-20}$-Alkylgruppen substituiert sein kann, und

$R^{11}$ $C_{1-20}$-Alkyl, $C_{6-10}$-Aryl, das durch eine oder zwei $C_{1-4}$-Alkylgruppen substituiert sein kann oder einen heterocyclischen Rest darstellt.

Gegenstand der Erfindung sind auch Salze von Verbindungen der Formel I mit Säuren oder Basen.

In den obigen Definitionen können Alkyl- und Alkenylgruppen, sofern nichts anderes angegeben ist, geradkettig oder verzweigt sein.

Stellt k die Zahl 2 dar, so können die Q gleiche oder verschiedene Bedeutung haben. Ist $R^2$ oder $R^3$ durch eine Gruppe $-COOR^4$ substituiert, so kann das $R^4$ in diesem Substituenten die gleiche oder eine andere Bedeutung als Gruppen $R^4$ in Resten Q haben.

Als Beispiele von geradkettigen $C_{1-5}$-Alkylgruppen W und/oder $R^{10}$ seien genannt: Methyl, Äthyl, n-Propyl, n-Butyl und n-Pentyl. Beispiele von $C_{1-12}$-Alkylgruppen in den Resten $R^1$ und Phenoxyacetylamino sind Methyl, Äthyl, n-Propyl, Isobutyl, n-Butyl, sek.-Butyl, tert.-Butyl, n-Pentyl, n-Hexyl, n-Heptyl, n-Octyl, 2-Äthylhexyl, 1,1,3,3-Tetramethylbutyl, n-Nonyl, n-Decyl und n-Dodecyl. Stellt $R^1$ $C_{2-12}$-Alkenyl dar, so handelt es sich z.B. um Vinyl, Prop-1-enyl, 1-Methylvinyl, But-1-enyl, Hexa-2,4-dienyl, Undec-10-enyl oder Dodec-1-enyl. Beispiele von $C_{3-8}$-Cycloalkylgruppen $R^1$ sind Cyclopropyl, Cyclobutyl, Cyclopentyl, Cyclohexyl, Cycloheptyl oder Cyclooctyl. Stellen $R^1$, $R^4$, $R^6$ oder $R^{11}$ $C_{6-10}$-Aryl dar, so handelt es sich z.B. um Phenyl oder Naphthyl.

Heterocyclische Reste $R^{11}$ weisen bevorzugt 3 bis 7 Ringglieder und ein oder mehrere Sauerstoff- Stickstoff- oder Schwefelatome auf. Als Beispiele seien genannt: Oxiran-, Azetidin-, Furan-, Thiophen-, Pyrrol-, Oxazol-, Isooxazol-, Thiazol-, Isothiazol-, Pyrazol-, Imidazol-, Triazol-, Oxadiazol-, Thiadiazol-, Thiatriazol-, Tetrazol-, Pyridin-, Pyrimidin-, Pyrazin-, Pyridazin-, Triazin- und Azepinreste.

Als stickstoffhaltige heterocyclische Reste X kommen vor allem solche mit 5 bis 7 Ringgliedern in Betracht, die ein oder zwei N-Atome und gegebenenfalls ein O- oder S-Atom enthalten, wie z.B. der Pyrrolidindion- und Piperidindionrest.

Beispiele von $C_{1-5}$-Alkylgruppen $R^2$ und $R^3$ sind Methyl, Äthyl, n-Propyl, Isopropyl, n-Butyl, sek.-Butyl, n-Pentyl und Neopentyl. Bedeuten $R^4$, $R^5$, $R^6$, $R^9$, $R^{10}$ oder $R^{11}$ $C_{1-20}$-Alkyl, so handelt es sich z.B. um Methyl, Äthyl, n-Propyl, Isopropyl, n-Butyl, sek.-Butyl, n-Pentyl, n-Hexyl, n-Heptyl, n-Octyl, 2-Äthylhexyl, n-Nonyl, n-Decyl, n-Dodecyl, n-Tetradecyl, n-Hexadecyl, n-Octadecyl oder n-Eicosyl. Beispiele von $C_{3-20}$-Alkenylgruppen $R^4$ oder $R^6$ sind: Prop-2-enyl, But-2-enyl, 3-Methylbut-2-enyl, Octadec-9-enyl oder Eicos-2-enyl.

Stellen $R^4$ oder $R^6$ $C_{3-12}$-Cycloalkyl dar, so kommen z.B. Cyclopropyl, Cyclobutyl, Cyclopentyl, Cyclohexyl, Cycloheptyl, Cyclooctyl, Cyclodecyl, Adamantyl oder Cyclododecyl in Betracht.

Als Beispiele von $C_{7-13}$-Aralkylgruppen $R^1$, $R^4$ oder $R^6$ seien genannt: Benzyl, Phenyläthyl, Benzhydryl oder Naphthylmethyl. Stellen $R^1$, $R^{11}$, $R^4$ oder $R^6$ $C_{6-10}$-Aryl dar, das durch eine oder zwei $C_{1-4}$-Alkylgruppen substituiert sein kann, oder sind durch $R^4$ und $R^5$ oder $R^7$ und $R^8$ zusammen mit dem Bindungs-N-Atom gebildete 5- oder 6gliedrige heterocyclische Ringe durch eine $C_{1-4}$-Alkylgruppen substituiert, so sind geeignete Alkylgruppen beispielsweise Methyl, Äthyl, n-Propyl, Isopropyl, n-Butyl, sek.-Butyl oder tert.-Butyl.

Geeignete $C_{1-4}$-Alkylgruppen $R^7$ oder $R^8$ sind z.B. Methyl, Äthyl, n-Propyl, Isopropyl, n-Butyl oder sek.-Butyl.

Bilden $R^9$ und $R^{10}$ zusammen $C_{2-3}$-Alkylen, das durch 1 oder mehrere $C_{1-20}$-Alkylgruppen substituiert sein kann, so handelt es sich z.B. um die folgenden Gruppen $-CH_2CH_2-$, $-CH_2CH_2CH_2-$, $-CH_2CH(CH_3)-$, $-CH_2CH(C_2H_5)-$, $-CH_2CH(C_{20}H_{41})-$, $-CH(CH_3)CH(CH_3)-$, $-CH(CH_3)C(CH_3)_2-$, $-C(CH_3)_2C(CH_3)_2-$, $-CH_2CH_2C(CH_3)_2-$ oder $-CH(CH_3)CH_2CH(CH_3)-$.

Bedeutet $R^4$ substituiertes $C_{6-10}$-Aryl, so kommen als Arylsubstituenten z.B. eine oder zwei $C_{1-4}$-Alkylgruppen, eine oder zwei $-CF_3-$, $-CN-$, $-CONH_2-$, $-COOCH_3-$, $-NO_2-$ oder $-OCH_3$-Gruppen oder ein oder zwei Halogenatome in Betracht. Stellt W Halogen dar oder ist die Arylgruppen $R^4$ durch Halogen substituiert, so handelt es sich dabei z.B. um $-F$, $-Cl$ oder $-Br$.

Als Salze von Verbindungen der Formel I kommen z.B. Alkalimetall- oder Erdalkalimetallsalze und Salze mit Kationen der Übergangselemente, Ammoniumkationen oder substituierten Ammoniumkationen in Betracht. Beispiele von Salzen von Verbindungen der Formel I, worin $Q-NR^7R^8$ darstellt, sind die Hydrochloride, Sulfate, p-Toluolsulfate, Maleate und Oxalate.

Die Substituenten W und Y können alle die Spektralabsorption des schwarzen Farbstoffs aus dem gekuppelten Resorcinol der Formel I beeinflussen. Dies ist hingegen beim Substituenten X nicht der Fall, weil diese Gruppe während der Kupplung abgespalten wird. Der Substituent X kann aber die Kupplungsgeschwindigkeit beeinflussen. In gewissen Fällen wird eine erhöhte Kupplungsgeschwindigkeit erreicht, wenn X nicht Wasserstoff, sondern Chlor oder Brom bedeutet. Die Gruppe $-SR^{11}$ kann als sogenannte entwicklungshemmende Gruppe bezeichnet werden. Unter Umständen kann es auch zweckmässig sein, dem photographischen Material einen DIR-Resorcinolkuppler (DIR=*development-inhibitor releasing*) dieser Art zuzugeben, um Inter- und Intrabildeffekte, wie eine Verstärkung des Bildrandes, zu erzeugen.

$R^2$ stellt bevorzugt Methyl dar. W ist vorzugsweise Wasserstoff, Chlor oder Brom, Methyl, Äthyl, Butyl, $-NHCOR^1$, worin $R^1$ $C_{1-6}$-Alkyl, $C_{2-6}$-Alkenyl, Cyclohexyl, Benzyl, Phenyl oder durch eine oder zwei Methyl oder Äthylgruppen substituiertes Phenyl bedeutet, oder W ferner Phenoxyacetylamino, das durch eine oder zwei gerad-

kettige oder verzweigte $C_{1-12}$-Alkylgruppen substituiert sein kann. Besonders bevorzugt stellt W Wasserstoff, Chlor, Brom, Methyl, $-NHCOCH_2C_6H_5$, $-NHCOC_6H_5$ oder eine gegebenenfalls durch 1 oder 2 $C_{1-12}$-Alkyl substituierte Phenoxyacetylaminogruppe dar.

Besonders bevorzugt sind Verbindungen der Formel I, worin n eine ganze Zahl von 1 bis 10, k die Zahl 1, $R^2$ Methyl, $R^3$ geradkettiges oder verzweigtes $C_{1-5}$-Alkyl, $Q-COOR^4$, $-CONR^4R^5$, $-OCOR^6$ oder $-NR^7R^8$, X Wasserstoff und W Wasserstoff, Chlor, Brom, Methyl, $-NHCOCH_2C_6H_5$, $-NHCOC_6H_5$ oder gegebenenfalls durch 1 oder 2 $C_{1-12}$-Alkyl substituierte Phenoxyacetylaminogruppe bedeuten. Ganz besonders bevorzugt sind Verbindungen der Formel I, worin n eine ganze Zahl von 3 bis 5, k die Zahl 1, $R^2$ und $R^3$ je Methyl, X Wasserstoff, W Wasserstoff, Methyl, $-NHCOCH_2C_6H_5-$, $-NHCOC_6H_5-$ oder eine gegebenenfalls durch ein oder zwei $C_{1-4}$-Alkyl substituierte Phenoxyacetylaminogruppe, und $Q-COOR^4$, $-CONR^4R^5$ oder $-NR^7R^8$ darstellen. Dabei können $R^4$, $R^5$, $R^6$, $R^7$ und $R^8$ die oben angegebene Bedeutung haben.

Gegenstand der Erfindung ist auch ein Verfahren zur Herstellung der Verbindungen der Formel I und Salzen davon mit Säuren oder Basen, indem man bei Temperaturen zwischen 20 und 150°C in Gegenwart einer Säure oder eines Friedel-Crafts-Katalysators eine Verbindung der Formel III

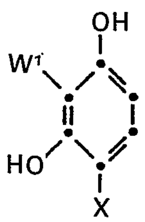

(III)

oder ein Salz davon, worin X die oben angegebene Bedeutung hat, $W^1$ Wasserstoff, n-Alkyl mit 1-5 C-Atomen, $-NH_2$, $-NHCOR^1$, $-COR^1$, $-NO_2$ oder Halogen ist, mit einem zur Einführung von Gruppen der Formel II befähigten funktionellen Alkylierungsmittel umsetzt, gegebenenfalls vorhandene Nitrogruppen $W^1$ zu Aminogruppen reduziert und Aminogruppen zu Gruppen $-NHCOR^1$ acyliert, gegebenenfalls in 2- und/oder 6-Stellung von Wasserstoff verschiedene Substituenten W und/oder X einführt, und/oder gegebenenfalls so erhaltene Verbindungen in die entsprechenden Salze mit Säuren oder Basen überführt. $R^1$ hat die oben angegebene Bedeutung.

Die Ausgangsverbindungen der Formel III sind bekannt und können nach an sich bekannten Methoden hergestellt werden.

Das Verhältnis von Verbindung der Formel III zu Alkylierungsmittel liegt zweckmässig zwischen 10:1 und insbesondere zwischen 5:1 bis 1:1. Überschüssige Verbindung der Formel III kann als Lösungsmittel dienen.

Gruppen $W=-NHCOR^1$ können dadurch in 2-Stellung eingeführt werden, dass man eine Verbindung der Formel I, worin W Wasserstoff ist, mit Salpetersäure oder einem anderen, für phenolische Verbindungen geeigneten Nitrierungsmittel nitriert, die Nitrogruppe mittels katalytischer Hydrierung oder anderer Reduktionsmittel zur Aminogruppe reduziert und dann die Aminogruppe mit zur Einführung des Restes $-COR^1$ befähigten N-Acylierungsmitteln behandelt. Als Acylierungsmittel kommen z.B. Ester, Säurehalogenide, Säureazide oder Säureanhydride, wie gemischte Anhydride aus einer Säure $R^1-COOH$ und einem Monoester der Kohlensäure, Pivalinsäure oder Trichloressigsäure oder freie Säuren in Gegenwart eines Kondensierungsmittels, z.B. Carbodiimide, in Betracht. Die genannten Reduktions- und Acylierungsreaktionen können gewünschtenfalls auch an Verbindungen der Formel III durchgeführt werden.

Halogen, Interhalogen und Gruppen $-SR^{11}$ können dadurch in Verbindungen der Formel I mit W und X=Wasserstoff eingeführt werden, indem man diese mit bis zu 3 mol einer Verbindung $W^2-Cl$ oder $W^2-Br$, worin $W^2$ Chlor, Brom oder $-SR^{11}$ bedeutet, zu einer Verbindung der Formel IV

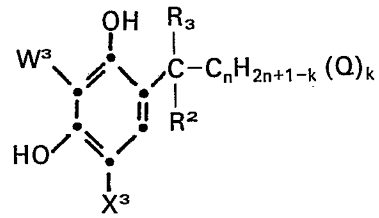

(IV)

umsetzt. Dabei haben $R^2$, $R^3$, Q, n und k die unter Formel I angegebene Bedeutung, eines von $W^3$ und $X^3$ ist Wasserstoff, Chlor, Brom oder $-SR^{11}$ und das andere Chlor, Brom oder $-SR^{11}$.

Als Beispiele von Verbindungen der Formel III seien genannt: Resorcinol, 2-Acetylresorcinol, 2-Methylresorcinol, 2-Äthylresorcinol, 2-n-Pentylresorcinol, 2-Nitroresorcinol, 2-Aminoresorcinol, 2-Acetamidoresorcinol, 2-Propionamidoresorcinol, 2-Benzamidoresorcinol, 2-Chlorresorcinol, 4-Chlorresorcinol, 2,4-Dichlor- und 2,4-Dibromresorcinol, 4-Methylthioresorcinol.

Die funktionellen Alkylierungsmittel, die mit den Verbindungen der Formel III umgesetzt werden, enthalten ein aktives Zentrum, z.B. eine olefinische Gruppe oder eine OH-Gruppe, die während der Alkylierungsreaktion eliminiert, umgewandelt oder umgelagert werden. Beispiele von geeigneten funktionellen Olefinen zur Umsetzung mit Verbindungen der Formel III sind: Methyl-5-methylhex-5-enoat, 4-Methoxycarbonyl-1-methylcyclohex-1-en, Dimethylphenylphosphonat, Diäthylphenylphosphonat, Citronellol, Citronellylacetat, 2-Acetamido-6-methyl-5-hepten, 2-Acetamido-6-methyl-6-hepten, 2-Methyl-2-propen-1-sulfonsäure, 2-Methyl-2-propen-1-sulfonamid, N-Methyl-2-methyl-2-propen-1-sulfonamid, N,N-Dimethyl-2-methyl-2-propen-1-sulfonamid und N,N-Diäthyl-2-methyl-2-propen-1-sulfonamid.

Als Beispiele für funktionelle Hydroxyverbindungen zur Alkylierung der Verbindungen der Formel III sind substituierte 11-Aminoundecanole der Formel V geeignet

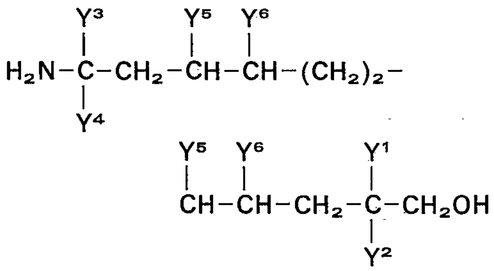

(V)

worin $Y^1$ und $Y^3$ unabhängig voneinander Wasserstoff oder $C_{1-8}$-Alkyl, $Y^2$ und $Y^4$ unabhängig voneinander $C_{1-8}$-Alkyl und $Y^5$ und $Y^6$ unabhängig voneinander Wasserstoff oder $C_{1-4}$-Alkyl bedeuten, oder aber insbesondere 2-Amino-6-hydroxy-6-methylheptan oder 11-Amino-2,2-,12-trimethyltridecan-1-ol.

Geeignete substituierte 11-Aminoundecanole der Formel V sind z.B. in der deutschen Offenlegungsschrift Nr. 2831299 beschrieben.

Gruppen Q in Verbindungen der Formel I können auch in andere Gruppen Q übergeführt werden. So können z.B. Gruppen Q=–COOH in entsprechende Ester übergeführt werden, oder Estergruppen Q können einer Umesterung unterworfen werden.

Als nicht limitierende Beispiele von erfindungsgemässen Verbindungen der Formel I seien genannt:

Methyl-5-(2',4'-dihydroxyphenyl)-5-methylhexanoat
Methyl-5-(2',4'-dihydroxy-5'-methylthiophenyl)-5-methylhexanoat
Methyl-5-(2',4'-dihydroxy-5'-n-pentylthiophenyl)-5-methylhexanoat
Methyl-5-(2',4'-dihydroxy-5'-phenylthiophenyl)-5-methylhexanoat
Methyl-5-[2',4'-dihydroxy-5'-(1-methyltetrazol-5-ylthio)phenyl]-5-methylhexanoat
Methyl-5-(3'-acetyl-2',4'-dihydroxyphenyl)-5-methylhexanoat
Methyl-5-(2',4'-dihydroxy-5'-succinimidophenyl)-5-methylhexanoat
Methyl-5-(2',4'-dihydroxy-3'-methylphenyl)-5-methylhexanoat
Methyl-5-(2',4'-dihydroxy-3'-n-pentylphenyl)-5-methylhexanoat
Methyl-5-(3'-acetamido-2',4'-dihydroxyphenyl)-5-methylhexanoat
Methyl-5-(2',4'-dihydroxy-3'-n-hexanoylaminophenyl)-5-methylhexanoat
Methyl-5-(2',4'-dihydroxy-3'-n-dodecanoylaminophenyl)-5-methylhexanoat
Methyl-5-(3'-acrylamido-2',4'-dihydroxyphenyl)-5-methylhexanoat
Methyl-5-(3'-cyclohexylcarbonylamino-2',4'-dihydroxyphenyl)-5-methylhexanoat
Methyl-5-(3'-chloro-2',4'-dihydroxyphenyl)-5-methylhexanoat
Methyl-5-(3'-bromo-2',4'-dihydroxyphenyl)-5-methylhexanoat
Methyl-5-(5'-chloro-2',4'-dihydroxyphenyl)-5-methylhexanoat
Methyl-5-(5'-bromo-2',4'-dihydroxyphenyl)-5-methylhexanoat
Methyl-5-(3',5'-dichloro-2',4'-dihydroxyphenyl)-5-methylhexanoat
Methyl-5-(3',5'-dibromo-2',4'-dihydroxyphenyl)-5-methylhexanoat
Methyl-5-(3'-benzamido-2',5'-dihydroxyphenyl)-5-methylhexanoat
2-n-Butoxyäthyl-5-(2',4'-dihydroxy-3'-phenylacetylaminophenyl)-5-methylhexanoat
Methyl-5-[2',4'-dihydroxy-3-(2",4"-dineopentylphenoxyacetylaminophenyl)-5-methylhexanoat
5-(2',4'-Dihydroxyphenyl)-5-methylhexansäure
Isopropyl-5-(2',4'-dihydroxyphenyl)-5-methylhexanoat
n-Hexyl-5-(2',4'-dihydroxyphenyl)-5-methylhexanoat
2-Äthylhexyl-5-(2',4'-dihydroxyphenyl)-5-methylhexanoat
n-Eicosyl-5-(2',4'-dihydroxyphenyl)-5-methylhexanoat
Allyl-5-(2',4'-dihydroxyphenyl)-5-methylhexanoat
5-(2',4'-Dihydroxyphenyl)-5-methylhexansäuredimethylamid
5-(2',4'-Dihydroxyphenyl)-5-methylhexansäurediäthylamid
5-(2',4'-Dihydroxyphenyl)-5-methylhexansäurediallylamid
5-(2',4'-Dihydroxyphenyl)-5-methylhexansäuredicyclohexylamid
5-(2',4'-Dihydroxyphenyl)-5-methylhexansäurebenzylamid
5-(2',4'-Dihydroxyphenyl)-5-methylhexansäure-n-butylanilid
N-[2-(2',4'-Dihydroxyphenyl)-5-methylhexanoyl]morpholin
7-(2',4'-Dihydroxyphenyl)-3,7-dimethyloctan-1-ol
7-(2',4'-Dihydroxyphenyl)-3,7-dimethyloct-1-ylacetat
7-(2',4'-Dihydroxyphenyl)-3,7-dimethyloct-1-ylpropionat
7-(2',4'-Dihydroxyphenyl)-3,7-dimethyloct-1-yl-n-hexanoat
7-(2',4'-Dihydroxyphenyl)-3,7-dimethyloct-1-yl-n-eicosanat
7-(2',4'-Dihydroxyphenyl)-3,7-dimethyloct-1-ylcrotonat
7-(2',4'-Dihydroxyphenyl)-3,7-dimethyloct-1-ylsorbat
7-(2',4'-Dihydroxyphenyl)-3,7-dimethyloct-1-ylphenylacetat
7-(2',4'-Dihydroxyphenyl)-3,7-dimethyloct-1-ylbenzoat
7-(2',4'-Dihydroxyphenyl)-3,7-dimethyloct-1-yloxycarbonylcyclohexan
4-Methoxycarbonyl-1-(2',4'-dihydroxyphenyl)-1-methylcyclohexan
2-Amino-6-(2',4'-dihydroxyphenyl)-6-methylheptan
3-Amino-12-(2',4'-dihydroxyphenyl)-2,12-dimethyltetradecan
3-Amino-13-(2',4'-dihydroxyphenyl)-2,12-dimethyltetradecan
2-N-Methylamino-6-(2',4'-dihydroxyphenyl)-6-methylheptan
2-N-n-Butylamino-6-(2',4'-dihydroxyphenyl)-6-methylpentan
2-Dimethylamino-6-(2',4'-dihydroxyphenyl)-6-methylheptan
2-Di-N-n-butylamino-6-(2',4'-dihydroxyphenyl)-6-methylheptan
6-(2',4'-Dihydroxyphenyl)-2-morpholino-6-methylheptan
2-Acetamido-6-(2',4'-dihydroxyphenyl)-6-methylheptan
2-Hexanoylamino-6-(2',4'-dihydroxyphenyl)-6-methylheptan
2-Benzamido-6-(2',4'-dihydroxyphenyl)-6-methylheptan
1-Acetamido-5-(2',4'-dihydroxyphenyl)-5-methylhexan
3-(2',4'-Dihydroxyphenyl)-3-methylbutanphosphonsäure
Dimethyl-3-(2',4'-dihydroxyphenyl)-3-methylbutanphosphonat
Cyclohexyl-5-(2',4'-dihydroxyphenyl)-5-methylhexanoat
Benzyl-5-(2',4'-dihydroxyphenyl)-5-methylhexanoat
Phenyl-5-(2',4'-dihydroxyphenyl)-5-methylhexanoat
p-Tolyl-5-(2',4'-dihydroxyphenyl)-5-methylhexanoat
5-(2',4'-Dihydroxyphenyl)-5-methylhexansäureamid
5-(2',4'-Dihydroxyphenyl)-5-methylhexansäuremethylamid
5-(2',4'-Dihydroxyphenyl)-5-methylhexansäureäthylamid
5-(2',4'-Dihydroxyphenyl)-5-methylhexansäure-n-hexylamid
5-(2',4'-Dihydroxyphenyl)-5-methylhexansäure-n-octylamid
5-(2',4'-Dihydroxyphenyl)-5-methylhexansäure-n-eicosylamid
Diäthyl-3-(2',4'-dihydroxyphenyl)-3-methylbutanphosphonat
Di-2-äthylhexyl-3-(2',4'-dihydroxyphenyl)-3-methylbutanphosphonat
n-Dodecylmethyl-3-(2',4'-dihydroxyphenyl)-3-methylbutanphosphonat
2-[3-(2',4'-Dihydroxyphenyl)-3-methylbut-1-yl]-4-methyl-1,3,2-dioxaphospholan
Äthyl-3-(2',4'-dihydroxyphenyl)-3-methylbutanmethylphosphinat
21-(2',4'-Dihydroxyphenyl)-21-methyldocosansäuremethylester
Methyl-5-(2',4'-dihydroxyphenyl)-5,7,7-trimethyloctanoat
Dimethyl-5-(2',4'-dihydroxyphenyl)-5-methylazelat
cis-4-Methoxycarbonyl-1-(2',4'-dihydroxyphenyl)-1-methylcyclohexan
trans-4-Methoxycarbonyl-1-(2',4'-dihydroxyphenyl)-1-methylcyclohexan
2-(2',4'-Dihydroxyphenyl)-2-methylpropansulfonsäure
N-Methyl-2-(2',4'-dihydroxyphenyl)-2-methylpropansulfonamid
N-n-Dodecyl-2-(2',4'-dihydroxyphenyl)-2-methylpropansulfonamid
N-n-Eicosyl-2-(2',4'-dihydroxyphenyl)-2-methylpropansulfonamid
N-Allyl-2-(2',4'-dihydroxyphenyl)-2-methylpropansulfonamid
N-Cyclohexyl-2-(2',4'-dihydroxyphenyl)-2-methylpropansulfonamid
N-Benzyl-2-(2',4'-dihydroxyphenyl)-2-methylpropansulfonamid
N-Phenyl-2-(2',4'-dihydroxyphenyl)-2-methylpropansulfonamid
N,N-Dimethyl-2-(2',4'-dihydroxyphenyl)-2-methylpropansulfonamid
N,N-Diäthyl-2-(2',4'-dihydroxyphenyl)-2-methylpropansulfonamid
N-[2-(2',4'-Dihydroxyphenyl)-2-methylpropansulfonyl]morpholin
1-Cyano-4-(2',4'-dihydroxyphenyl)-4-methylpentan
5-(2',4'-Dihydroxyphenyl)-5-methylazelainsäure
Diäthyl-5-(2',4'-dihydroxyphenyl)-5-methylazelat
Di-n-propyl-5-(2',4'-dihydroxyphenyl)-5-methylazelat

Die Verbindungen der Formel I stellen wertvolle Zwischenprodukte für photographische Materialien, insbesondere auf Silberhalogenid-Basis, dar, enthaltend mindestens eine auf einem Trägermaterial ausgetragene Silberhalogenidemulsionsschicht. Derartige Verbindungen der Formel I werden insbesondere in der Silberhalogenidemulsionsschicht eingebaut, können aber auch in einer Schicht vorhanden sein, welche in Kontakt mit mindestens einer Silberhalogenidemulsionsschicht steht.

Die photographischen Materialien, enthaltend eine Verbindung der Formel I, werden nach Belichtung mit einem Farbentwicklungsverfahren aufgearbeitet, wobei als Farbentwicklungsmittel primäre aromatische Amine bekannter Strukturen verwendet werden.

Als Trägermaterialien können beliebige Träger verwendet werden, die in der Photographie üblich sind.

Mit den Verbindungen der Formel I wird eine wertvolle Möglichkeit zur Einführung einer Vielzahl von funktionellen Alkylresten mit optimaler photographischer Wirkung geschaffen. Beispielsweise kann die Polarität und/oder der Ballast in den Resorcinolen der Formel I so eingestellt werden, dass dadurch eine wirksame Kontrolle der Löslichkeit, Verträglichkeit, Beweglichkeit/Unbeweglichkeit in photographischen Systemen ermöglicht wird. Derartige Eigenschaften machen die Verbindungen der Formel I als wertvolle Zwischenprodukte zur Herstellung von komplizierten photographischen Materialien sowie als schwarze Farbkuppler interessant.

In den folgenden Beispielen bedeuten Teile Gewichtsteile und Prozente Gewichtsprozente. Die Analysenwerte stellen Gewichtsprozente dar.

*Beispiel 1:*

110 Teile Resorcinol, 28,4 Teile Methyl-5-methylhex-5-enoat (hergestellt gemäss US-PS Nr. 3783136) und 5,0 Teile Aktiverde Fulmont® 237 werden bei 125-130°C 18 h gerührt. Das abgekühlte Reaktionsgemisch wird mit Diäthyläther verdünnt, durch Filtrieren vom Katalysator befreit und der Diäthyläther abgezogen. Die Destillation des zurückbleibenden Öls ergibt 91,0 Teile einer bis zu 190°C/10 bar siedenden Fraktion, die zur Hauptsache aus Resorcinol besteht, sowie 30 Teile einer bei 186-194°C/0,2 bar siedenden Fraktion. Aus dieser letzteren Fraktion erhält man nach Verdünnen mit Petroläther (Siedebereich 40-60°C), der etwas Diäthyläther enthält, das Methyl-5-(2',4'-dihydroxyphenyl)-5-methylhexanoat der Formel

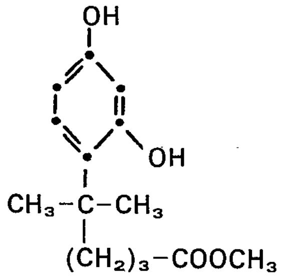

Schmelzpunkt = Fp. 93-96°C.

*Analyse* für $C_{14}H_{20}O_4$:

| | | |
|---|---|---|
| Berechnet: | C 66,65 | H 7,99% |
| Gefunden: | C 66,34 | H 8,13% |

*Beispiel 2:*

Auf die in Beispiel 1 beschriebene Weise werden 55 Teile Resorcinol, 15,4 Teile 4-Methoxycarbonyl-1-methylcyclohex-1-en (hergestellt gemäss Kojima *et al.*, „J. Org. Chem.", *36*, 924 [1971]) und 5,0 Tile Aktiverde Fulmont® 237 umgesetzt und aufgearbeitet. Die Destillation ergibt 42,6 Teile zurückgewonnenes Resorcinol und 15,4 Teile cis-/trans-4-Methoxycarbonyl-1-(2',4'-dihydroxyphenyl))-1-methylcyclohexan; Siedepunkt 218°C/0,3 bar.

*Analyse* für $C_{15}H_{20}O_4$:

| | | |
|---|---|---|
| Berechnet: | C 68,16 | H 7,63% |
| Gefunden: | C 67,93 | H 7,77% |

*Beispiel 3:*

Nach der in Beispiel 1 beschriebenen Arbeitsweise erhält man aus 73 Teilen Resorcinol, 52 Teilen Citronellol und 60 Teilen Aktiverde Fulmont® 237 36 Teile 7-(2',4'-Dihydroxyphenyl)-3,7-dimethyloctan-1-ol; Siedepunkt 210°C/0,2 bar.

*Analyse* für $C_{16}H_{26}O_3$:

| | | |
|---|---|---|
| Berechnet: | C 72,14 | H 9,84% |
| Gefunden: | C 72,56 | H 10,40% |

*Beispiel 4:*

11,0 Teile Resorcinol, 10,0 Teile Citronellylacetat und 0,5 Teile p-Toluolsulfonsäure werden 45 h auf 115°C erhitzt. Das Reaktionsgemisch wird dann in Diäthyläther aufgenommen, mit Natriumbicarbonatlösung, dann mit Wasser gewaschen und dann eingedampft. Durch Destillation des zurückbleibenden Öls erhält man 3,9 Teile 7-(2',4'-Dihydroxyphenyl)-3,7-dimethyloct-1-ylacetat; Siedepunkt 215°C/0,2 bar.

*Analyse* für $C_{18}H_{28}O_4$:

| | | |
|---|---|---|
| Berechnet: | C 70,09 | H 9,15% |
| Gefunden: | C 70,44 | H 9,20% |

*Beispiel 5:*

a) Zu 36,3 Teilen 2-Amino-6-hydroxy-6-methylheptan (J. Doeuvre und J. Poizat, „Compt. rend.", *224*, 286-8 [1947]) in 100 Teilen Diäthyläther gibt man unter Rühren 25,5 Teile Acetanhydrid. Dabei wird die Temperatur bei 20°C gehalten. Nach Beendigung der Zugabe wird das Reaktionsgemisch noch während 30 min gerührt. Flüchtige Bestandteile werden unter vermindertem Druck entfernt. Die Destillation des zurückbleibenden Öls ergibt 35,4 Teile 2-Acetamido-6-hydroxy-6-methylheptan; Siedepunkt 196-198°C/12 bar.

*Analyse* für $C_{10}H_{21}NO_2$:

| | | | |
|---|---|---|---|
| Berechnet: | C 64,13 | H 11,30 | N 7,48% |
| Gefunden: | C 63,10 | H 11,71 | N 7,66% |

b) 11,0 Teile Resorcinol, 7,5 Teile 2-Acetamido-6-hydroxy-6-methylheptan und 0,5 Teile p-Toluolsulfonsäure werden in einem Carius-Glasrohr 3 d bei 120°C gehalten. Nach dem Verdünnen mit Diäthyläther wird das Reaktionsgemisch mit Natriumbicarbonatlösung und dann mit Wasser gewaschen und verdampft. Die Destillation des zurückbleibenden Öls ergibt nach einem Vorlauf von 5,1 Teilen Resorcinol 6,3 Teile 2-Acetamido-6-(2',4'-dihydroxyphenyl)-6-methylheptan; Siedepunkt 234-240°C/0,2 bar. Diese Fraktion kristallisiert nach dem Verdünnen mit Diäthyläther zu einem weissen Feststoff vom Schmelzpunkt = Fp. 197-200°C.

*Analyse* für $C_{16}H_{25}NO_3$:

| | | | |
|---|---|---|---|
| Berechnet: | C 68,79 | H 9,02 | N 5,01% |
| Gefunden: | C 68,59 | H 9,03 | N 4,95% |

*Beispiel 6:*

0,64 Teile 5-Mercapto-1-methyltetrazol werden in 17 Teilen 1,1,1-Trichloräthan suspendiert. Dann wird Chlor durch dieses Gemisch geleitet, bis vollständige Lösung eintritt. Dann wird unter vermindertem Druck zur Trockne eingedampft, mit zwei weiteren Portionen Trichloräthan behandelt und wieder zur Trockne eingedampft. Der Rückstand wird in 19 Teilen Trichloräthan aufgenommen und unter Stickstoff sowie unter Feuchtigkeitsausschluss im Verlaufe von 15 min zu einer rückflussierenden Lösung von 1,26 Teilen Methyl-5-(2',4'-dihydroxyphenyl)-5-methylhexanoat (hergestellt nach Beispiel 1) in 19 Teilen Trichloräthan zugetropft. Das Reaktionsgemisch wird 8 h am Rückfluss gehalten, dann abgekühlt, durch Filtrieren von geringen Mengen Feststoff befreit und zur Trockne eingedampft. Man erhält ein nichtflüchtiges Öl, das durch Reinigung mittels Dünnschichtchromatographie (Kieselgel/1:1-

Äthylacetat/Cyclohexan) gläsernes Methyl-5-[2',4'-dihydroxy-5'-(1"-methyltetrazol-5"-yl)-thiophenyl]-5-methylhexanoat der Formel

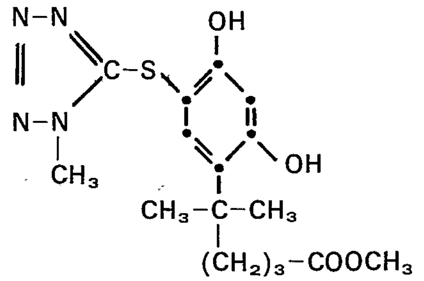

ergibt.

*Beispiel 7:*

5,5 Teile Methyl-5-(2',4'-dihydroxyphenyl)-5-methylhexanoat, 50 Teile n-Hexanol und 0,5 Teile p-Toluolsulfonsäure werden 6 h auf einem Dampfbad erhitzt. Überschüssiges Hexanol wird dann unter vermindertem Druck entfernt. Das zurückbleibende Öl wird in Diäthyläther aufgenommen, mit Natriumbicarbonatlösung und Wasser gewaschen und eingedampft. Durch Kurzwegdestillation des resultierenden Öls bei 0,7 mbar erhält man 5,8 Teile n-Hexyl-(2',4'-dihydroxyphenyl)-5-methylhexanoat der Formel

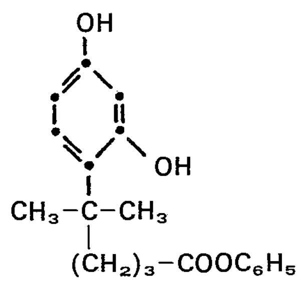

*Analyse* für $C_{19}H_{30}O_4$:

| | | |
|---|---|---|
| Berechnet: | C 70,77 | H 9,38% |
| Gefunden: | C 70,69 | H 9,71% |

*Beispiel 8:*

10,0 Teile 2-Methylresorcinol, 5,7 Teile Methyl-hex-5-enoat und 2,5 Teile Aktiverde Fulmont® 237 werden wie in Beispiel 1 beschrieben umgesetzt und aufgearbeitet. Die Destillation ergibt Methyl-5- (2',4'-dihydroxy-3'-methylphenyl)-5-methylhexanoat; Siedepunkt 196°C/0,7 bar.

*Analyse* für $C_{15}H_{22}O_4$:

| | | |
|---|---|---|
| Berechnet: | C 67,65 | H 8,33% |
| Gefunden: | C 67,95 | H 8,57% |

*Beispiel 9:*

11,0 Teile Resorcinol, 12,2 Teile 2-Hexanoylamino-6-hydroxy-6-methylheptan und 0,5 Teile p-Toluolsulfonsäure werden 4 d in einem Carius-Glasrohr gehalten. Dann wird das Reaktionsgemisch in 500 Teile Wasser gegossen und 30 min auf einem Dampfbad gerührt. Nach dem Entfernen des Wassers durch Dekantieren wird der obige Waschvorgang zweimal wiederholt. Dann wird das zurückbleibende Öl in Diäthyläther aufgenommen. Die Ätherlösung wird getrocknet und unter vermindertem Druck (16 mbar) auf einem Rotationsverdampfer bei 100°C abgezogen. Man erhält 2-Hexanoylamino-6-(2',4'-dihydroxyphenyl))-6-methylheptan als bernsteifarbenes Öl.

*Analyse* für $C_{20}H_{33}NO_3$:

| | | | |
|---|---|---|---|
| Berechnet: | C 71,60 | H 9,92 | N 4,18% |
| Gefunden: | C 71,19 | H 10,29 | N 4,02% |

*Beispiele 10-15:*

2,5 Teile Methyl-5-(2',4'-dihydroxyphenyl)-5-methylhexanoat und 5,0 Teile n-Octylamin werden in einem Carius-Glasrohr 3 d bei 120°C gehalten. Das abgekühlte Reaktionsgemisch wird in Diäthyläther aufgenommen, zuerst mit verdünnter HCl von überschüssigem Octylamin befreit und dann mit Wasser gewaschen. Nach dem Entfernen des Diäthyläthers und Verdünnen des zurückgebliebenen Öls mit Petroläther (Siedebereich 40-60°C), der etwas Diäthyläther enthält, erhält man 5-(2',4'-Dihydroxyphenyl)-5-methylhexansäure-n-octylamid; Schmelzpunkt = Fp. 110-113°C.

*Analyse* für $C_{21}H_{35}NO_3$:

| | | | |
|---|---|---|---|
| Berechnet: | C 72,17 | H 10,09 | N 4,01% |
| Gefunden: | C 71,99 | H 9,99 | N 3,90% |

Auf analoge Weise werden die folgenden Verbindungen hergestellt

*Beispiel 11:*

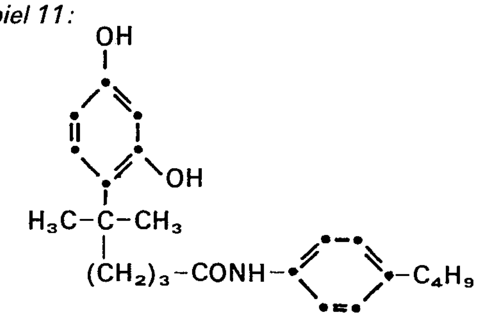

*Beispiel 12:*

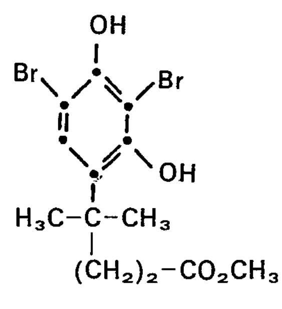

*Beispiel 13:*

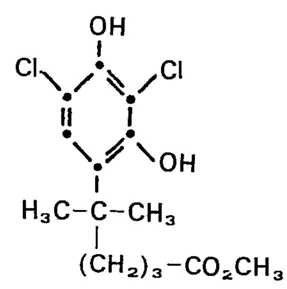

*Beispiel 14:*

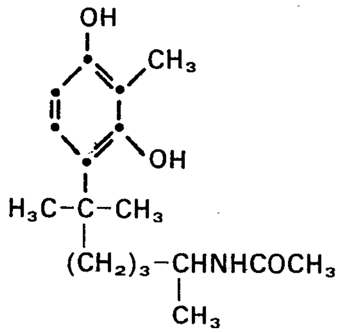

*Beispiel 15:*

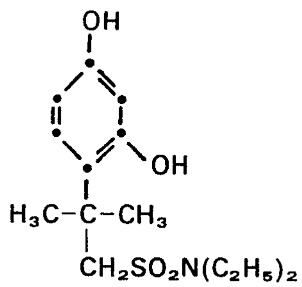

*Beispiel 16:*

5,0 Teile Methyl-5-(2',4'-dihydroxyphenyl)-5-methylhexanoat, 50 Teile 2-Äthylhexanol und 0,5 Teile p-Toluolsulfonsäure werden auf einem Dampfbad 18 h erhitzt. Dann wird das Reaktionsgemisch mit Diäthyläther verdünnt, mit Natriumbicarbonatlösung und Wasser gewaschen und eingedampft. Nach dem Abziehen des überschüssigen 2-Äthylhexanols auf einem Rotationsverdampfer bei 100°C/0,13 mbar erhält man aus dem Rückstand 2-Äthylhexyl-5-(2',4'-dihydroxyphenyl)-5-methylhexanoat als viskose bernsteinfarbene Flüssigkeit.

*Analyse* für $C_{21}H_{34}O_4$:

| | | |
|---|---|---|
| Berechnet: | C 71,86 | H 9,78% |
| Gefunden: | C 71,94 | H 9,89% |

*Beispiel 17:*

Analog der in Beispiel 16 beschriebenen Arbeitsweise, aber unter Verwendung von n-Dodecanol anstelle von 2-Äthylhexanol erhält man n-Dodecyl-5-(2',4'-dihydroxyphenyl)-5-methylhexanoat in Form eines bernsteinfarbenen Syrups.

*Analyse* für $C_{25}H_{42}O_4$:

| | | |
|---|---|---|
| Berechnet: | C 73,85 | H 10,41% |
| Gefunden: | C 74,24 | H 10,38% |

*Beispiel 18:*

55,0 Teile Resorcinol, 17,8 Teile Dimethylprenylphosphonat und 5,0 Teile Aktiverde Fulmont® 237 werden bei 125°C 18 h gerührt. Das abgekühlte Reaktionsgemisch wird mit Diäthyläther verdünnt, durch Filtrieren vom Katalysator befreit und in 500 Teile Wasser gegossen. Das ausgefallene Öl wird dann durch Dekantieren mit Wasser gewaschen und mit Diäthyläther extrahiert. Nach weiterem Waschen mit Natriumbicarbonatlösung, dann mit Wasser und Einengen erhält man aus der Ätherlösung Dimethyl-3-(2',4'-dihydroxyphenyl)-3-methylbutanphosphonat; Schmelzpunkt = Fp. 142-143°C.

*Analyse* für $C_{13}H_{21}O_5P$:

| | | | |
|---|---|---|---|
| Berechnet: | C 54,16 | H 7,34 | P 10,75% |
| Gefunden: | C 53,97 | H 7,33 | P 10,94% |

ℜ: BE12 J: 31-6 PTI31-6c 69066

*Beispiel 19:*

Analog der in Beispiel 18 beschriebenen Arbeitsweise, aber unter Verwendung von 20,6 Teilen Diäthylprenylphosphonat anstelle von Dimethylprenylphosphonat, erhält man nach Kristallisation aus Äthanol/Äthyläther Diäthyl-3-(2',4'-dihydroxyphenyl)-3-methylbutanphosphonat-monohydrat; Schmelzpunkt = Fp. 94-96°C.

*Analyse* für $C_{15}H_{25}PO_5 \cdot H_2O$:

| | | | |
|---|---|---|---|
| Berechnet: | C 53,88 | H 8,14 | P 9,26% |
| Gefunden: | C 53,74 | H 8,21 | P 9,47% |

*Beispiel 20:*

5,0 Teile Methyl-5-(2',4'-dihydroxyphenyl)-5-methylhexanoat und 10 Teile Di-n-butylamin werden 48 h bei 160°C in einem Carius-Glasrohr gehalten. Nach Aufarbeitung gemäss Beispiel 10 und nach Kurzwegdestillation bei 0,3 mbar erhält man 5-(2',4'-Dihydroxyphenyl)-5-methylhexansäure-N,N-di-n-butylamid in Form eines viskosen bernsteinfarbenen Öls.

*Analyse* für $C_{21}H_{35}NO_3$:

| | | | |
|---|---|---|---|
| Berechnet: | C 72,17 | H 10,09 | N 4,01% |
| Gefunden: | C 72,50 | H 9,61 | N 3,66% |

*Beispiel 21:*

1,5 Teile 2-Acetylresorcinol, 1,4 Teile Methyl-5-methylhex-5-enoat und 0,1 Teile para-Toluolsulfonsäure werden 48 h bei 100°C in einem Carius-Glasrohr gehalten. Die Reaktionsmischung wird danach vom Restkatalysator durch Waschen befreit. Nach Reinigen durch Dünnschichtchromatographie über Kieselgel und Kristallisation aus Petroläther (Siedebereich 40-60°C) wird Methyl-5-(3'-acetyl-2',4'-dihydroxyphenyl)-5-methylhexanoat isoliert; Schmelzpunkt = Fp. 94-97°C.

*Analyse* für $C_{16}H_{22}O_5$:

| | | |
|---|---|---|
| Berechnet: | C 65,29 | H 7,53% |
| Gefunden: | C 65,39 | H 7,30% |

*Beispiel 22:*

*Formulierung eines Resorcinols als ölige Dispersion*

A. 1 g des gemäss Beispiel 1 erhaltenen Resorcinols wird in 1 g isopropyliertem Phenylphosphat und 1 g Äthylacetat durch Erhitzen auf Rückfluss gelöst und dann auf 50°C abgekühlt.

B. 1 ml 10%ig (Vol./Vol. in $H_2O$) sulfoniertes PEO (Polyäthylenoxid, Netzmittel) und 3 ml destilliertes Wasser werden bei ca. 50°C zu 8 g 10%iger deionisierter Gelatine (Gew.-% in Wasser) gegeben.

A und B werden dann durch Rühren von Hand vermischt und während ca. 30 s in einem Ultraschallmischgerät dispergiert.

*Formulierung für Beschichtungen*

0,45 g der obigen Kupplerdispersion werden zu 1,70 g einer 10%igen deionisierten Gellösung und 0,54 g Silberjodid/Bromid-Emulsion (9,2% Jodid) gegeben.

Die Emulsion enthält 162 g Silber und 100 g Gel in 1467 g Totalgewicht. Der Formulierung wird ein Triazinhärter in einer Menge von 20 mol/$10^5$ g Gel zugegeben. Nach ausreichendem Mischen wird die Beschichtungsformulierung von Hand auf eine 2,4-$dm^2$-Polyesterbasis (5 Tausendstel dick) aufgetragen, die während des Beschichtens bei 40°C gehalten wird. Das Gewicht von Kuppler und Silberbeschichtung beträgt ca. 25 mg/$dm^2$ mit einem Gewicht der Gelbeschichtung von ca. 80 mg/$dm^2$. Die Beschichtung wird getrocknet und dann 12 h bei 45°C und 65% relativer Luftfeuchtigkeit gehalten. Die Beschichtungen werden 10 s mit weissem Licht bestrahlt und wie folgt bei 38°C weiterbehandelt:

*Entwicklung* 5 min: 0,37 g $K_2CO_3$, 1,5 ml $K_2SO_3$ (65%ige Lösung), 1,05 g KBr, 6,0 ml Diäthylentriaminpentaessigsäure (37%ige Lösung), 2 g Hydroxylaminsulfat, 1 ml $H_2SO_4$ (5N), 2,40 g 4-[N-Äthyl-N-(2'-Hydroxyäthyl)amino]-2-methylanilinhydrosulfat, 1,33 g $Na_2S_2O_5$, 0,94 ml 80%ige Essigsäure und Wasser bis zu 1 l; pH 10,20.

*Bleichen* 6,5 min: 150 g Ammoniumbromid, 112 g Ammoniumsalz von Fe(III)-Äthylendiamintetraessigsäure, 2,5 g Äthylendiamintetraessigsäure, 35 g Natriumnitrat, 10 ml Eisessig, Wasser bis zu 1 l; pH 6,0+0,2.

*Fixieren* 6,5 min: 130 g Ammoniumthiosulfat, 1,25 g Äthylendiamintetraessigsäuredinatriumsalz, 12 g Natriummetabisulfit, 2 g Natriumhydroxid; pH 6,5+0,2.

3 min Waschen bei 38°C.

Das Absorptionsspektrum zeigt, dass eine tief schwarze Farbe erzielt wird, die über das ganze sichtbare Spektrum sehr gut absorbiert.

Charakteristische Werte des Absorptionsspektrums sind in der folgenden Tabelle zusammengefasst (optische Dichte bei bestimmten Wellenlängen in Nonometern).

| Wellenlänge (nm) | optische Dichte |
|---|---|
| 345 | 1,5 |
| 350 | 1,35 |
| 400 | 0,95 |
| 450 | 0,98 |
| 480 | 1,02 |
| 500 | 0,98 |
| 550 | 0,82 |
| 600 | 0,72 |
| 650 | 0,63 |
| 700 | 0,53 |

## Patentansprüche

1. Verbindungen der Formel (I)

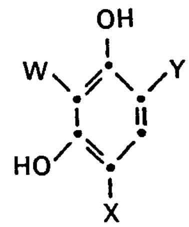

(I)

worin

W Wasserstoff, Halogen, n-Alkyl mit 1-5 C-Atomen, $-NHCOR^1$, $-COR^1$ oder eine gegebenenfalls durch eine oder zwei $C_{1-12}$ Alkylgruppen substituierte Phenoxyacetylaminogruppe,

X Wasserstoff, Chlor, Brom, $-SR^{11}$ oder den Rest eines über ein Ring-N-Atom gebundenen N-Heterocyclus,

Y eine Gruppe der Formel (II)

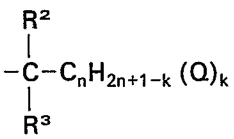

(II) und

Q $-COOR^4$, $-CONR^4R^5$, $-OR^5$, $-OCOR^6$, $-NR^7R^8$, $-PO(OR^9)[O]_xR^{10}$ mit x=0 oder 1, $-SO_2OH$, $-SO_2NR^4R^5$ oder $-CN$ bedeuten,

n eine ganze Zahl von 1 bis 20 ist,

k 1 oder 2 darstellt,

$R^1$ $C_{1-12}$-Alkyl, $C_{2-12}$-Alkenyl, $C_{3-8}$-Cycloalkyl, $C_{7-13}$-Aralkyl oder $C_{6-10}$-Aryl bedeutet, das durch eine oder zwei $C_{1-4}$-Alkylgruppen substituiert sein kann,

$R^2$ und $R^3$ unabhängig voneinander $C_{1-5}$-Alkyl darstellen, wobei bei $Q=-COOR^4R^2$ oder $R^3$ durch eine oder zwei $-COOR^4$-Gruppen substituiert sein kann, oder mindestens eines von $R^2$ und $R^3$ so an den Rest $-C_nH_{2n+1-k}$ gebunden ist, dass ein durch $-(COOR^4)_k$ substituierter $C_{5-12}$-Cycloalkylenrest entsteht, wobei die Gruppen $R^4$ gleiche oder verschiedene Bedeutungen haben können,

$R^4$ Wasserstoff, $C_{1-20}$-Alkyl, das durch ein oder mehrere O-Atome unterbrochen sein kann, $C_{3-20}$-Alkenyl, $C_{3-12}$-Cycloalkyl, $C_{7-13}$-Aralkyl oder gegebenenfalls substituiertes $C_{6-10}$-Aryl, und

$R^5$ Wasserstoff oder $C_{1-20}$-Alkyl bedeuten, oder

$R^4$ und $R^5$ zusammen mit dem N-Atom, an das sie gebunden sind, einen 5- oder 6gliedrigen heterocyclischen Ring bilden, der durch eine $C_{1-4}$-Alkylgruppe substituiert sein kann,

$R^6$ Wasserstoff, $C_{1-20}$-Alkyl oder $C_{3-20}$-Alkenyl, $C_{3-12}$-Cycloalkyl, $C_{7-13}$-Aralkyl oder gegebenen-

falls durch eine oder zwei $C_{1-4}$-Alkylgruppen substituiertes $C_{6-10}$-Aryl,

$R^7$ Wasserstoff oder $C_{1-4}$-Alkyl, und

$R^8$ Wasserstoff, $C_{1-4}$-Alkyl oder $-COR^4$ darstellen, oder

$R^7$ und $R^8$ zusammen mit dem N-Atom, an das sie gebunden sind, einen 5- oder 6gliedrigen heterocyclischen Ring bilden, der durch eine $C_{1-4}$-Alkylgruppe substituiert sein kann,

$R^9$ Wasserstoff oder $C_{1-20}$-Alkyl,

$R^{10}$ bei x=1 Wasserstoff oder $C_{1-20}$-Alkyl und bei x=0 $C_{1-5}$-Alkyl bedeuten oder $R^9$ und $R^{10}$ zusammen $C_{2-3}$-Alkylen bilden, das durch 1 oder mehrere $C_{1-20}$-Alkylgruppen substituiert sein kann, und

$R^{11}$ $C_{1-20}$-Alkyl, $C_{6-10}$-Aryl, das durch eine oder zwei $C_{1-4}$-Alkylgruppen substituiert sein kann oder einen heterocyclischen Rest darstellt,

sowie Salze von Verbindungen der Formel I mit Säuren oder Basen.

2. Verbindungen der Formel (I) nach Anspruch 1, worin W Wasserstoff, Halogen, n-Alkyl mit 1-5 C-Atomen, $-NHCOR^1$ oder $-COR^1$ bedeutet, und X, Y die im Anspruch 1 angegebene Bedeutung haben.

3. Verbindungen der Formel (I) nach Anspruch 1, worin $R^2$ Methyl darstellt.

4. Verbindungen der Formel (I) nach Anspruch 1, worin W Wasserstoff, Chlor, Brom, Methyl, Äthyl, Butyl, $-NHCOOR^1$ oder gegebenenfalls durch eine oder zwei $C_{1-12}$-Alkylgruppen substituiertes Phenoxyacetylamino, und $R^1$ $C_{1-6}$-Alkyl, $C_{2-6}$-Alkenyl, Cyclohexyl, Benzyl, Phenyl oder durch eine oder zwei Methyl- oder Äthylgruppen substituiertes Phenyl bedeuten.

5. Verbindungen der Formel (I) nach Anspruch 1, worin Wasserstoff, Chlor, Brom, Methyl, $-NHCOCH_2C_6H_5-$, $-NHCOC_6H_5$ oder gegebenenfalls durch eine oder zwei $C_{1-12}$ Alkylgruppen substituiertes Phenoxyacetylamino bedeutet.

6. Verbindungen der Formel (I) nach Anspruch 1, worin n eine ganze Zahl von 1 bis 10, k die Zahl 1, $R^2$ Methyl, $R^3$ geradkettiges oder verzweigtes $C_{1-5}$-Alkyl, $Q-COOR^4$, $-CONR^4R^5$, $-OR^5$, $-OCOR^6$ oder $-NR^7R^8$, X Wasserstoff und W Wasserstoff, Chor, Brom, Methyl, $-NHCOCH_2C_6H_5$ oder $-NHCOC_6H_5$ bedeuten, wobei $R^4$, $R^5$, $R^6$, $R^7$ und $R^8$ die im Anspruch 1 angegebene Bedeutung haben.

7. Verbindungen der Formel (I) nach Anspruch 1, worin n eine ganze Zahl von 3 bis 5, k die Zahl 1, $R^2$ und $R^3$ je Methyl, X Wasserstoff, Q $-COOR^4$, $-CONR^4R^5$ oder $-NR^7R^8$, W Wasserstoff, Methyl, $-NHCOCH_2C_6H_5$, $-NH-COC_6H_5$ oder gegebenenfalls durch eine oder zwei $C_{1-12}$-Alkylgruppen substituiertes Phenoxyacetylamino darstellen, wobei $R^4$, $R^5$, $R^7$ und $R^8$ die im Anspruch 1 angegebene Bedeutung haben.

8. Verfahren zur Herstellung von Verbindungen der Formel (I) und Salzen davon mit Säuren oder Basen nach Anspruch 1, dadurch gekennzeichnet, dass man bei Temperaturen zwischen 20 und 150°C in Gegenwart einer Säure oder eines Friedel-Crafts-Katalysators eine Verbindung der Formel (III)

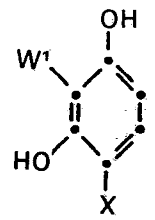

(III)

oder ein Salz davon mit einer Säure oder Base, worin X die im Anspruch 1 angegebene Bedeutung hat, $W^1$ Wasserstoff, n-Alkyl mit 1-5 C-Atomen, $-NH_2$, $-NHCOR^1$, $-COR^1$, $-NO_2$ oder Halogen ist, worin $R^1$ die im Anspruch 1 angegebene Bedeutung hat, mit einem zur Einführung von Gruppen der Formel II befähigten funktionellen Alkylierungsmittel umsetzt, gegebenenfalls vorhandene Nitrogruppen $W^1$ zu Aminogruppen reduziert und Aminogruppen zu Gruppen $-NHCOR^1$ acyliert, gegebenenfalls in 2- und/oder 6-Stellung von Wasserstoff verschiedene Substituenten W und/oder X einführt, und/oder gegebenenfalls so erhaltene Verbindungen der Formel I in die entsprechenden Salze mit Säuren oder Basen überführt.

9. Verfahren nach Anspruch 8, dadurch gekennzeichnet, dass man die Verbindungen der Formel (III) und das Alkylierungsmittel in einem Verhältnis von 5:1 bis 1:1 einsetzt.

10. Verfahren nach Anspruch 8, dadurch gekennzeichnet, dass eine Gruppe Q in eine andersartige Gruppe Q übergeführt wird.

## Claims

1. A compound of the Formula (I)

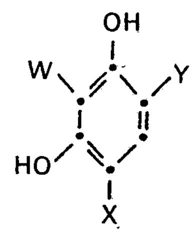

(I)

wherein

W is hydrogen, halogen, n-alkyl having from 1 to 5 carbon atoms, $-NHCOR^1$ or $-COR^1$ or is phenoxyacetylamino which is unsubstituted or substituted by one or two $C_{1-12}$ alkyl groups,

X is hydrogen, chlorine, bromine, $-SR^{11}$, or a nitrogen-containing heterocyclic residue attached at a ring nitrogen atom,

Y is a group of the formula (II)

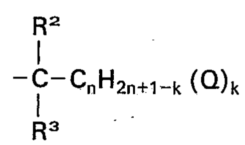

(II)

Q is $COOR^4$ or $-CONR^4R^5$, $-OR^5$, $-COR^6$, $-NR^7R^8$, $-PO(OR^9)[O]_xR^{10}$, in which x is 0 or 1, $-SO_2OH$, $-SO_2NR^4R^5$ or $-CN$,

n is a value from 1 to 20,

k is 1 or 2,

$R^1$ is $C_1$-$C_{12}$ alkyl, $C_2$-$C_{12}$alkenyl, $C_3$-$C_8$cycloalkyl, $C_7$-$C_{13}$aralkyl or $C_6$-$C_{10}$aryl which

can be substituted by one or two $C_1$-$C_4$alkyl groups,

$R^2$ and $R^3$ are each independently $C_1$-$C_5$alkyl, with the proviso that, if Q is $-COOR^4$, $R^2$ or $R^3$ can be substituted by one or two $-COOR^4$ groups, or at least one of $R^2$ and $R^3$ is attached to the $C_nH_{2n+1-k}$ radical so as to form a $C_5$-$C_{12}$ cycloalkenyl radical substituted by $-(COOR^4)_k$, in which the groups $R^4$ may be the same or different,

$R^4$ is hydrogen, $C_1$-$C_{20}$alkyl which can be interrupted by one or more oxygen atoms, or is $C_3$-$C_{20}$alkenyl, $C_3$-$C_{12}$cycloalkyl, $C_7$-$C_{13}$aralkyl or unsubstituted or substituted $C_6$-$C_{10}$aryl, and

$R^5$ is hydrogen or $C_1$-$C_{20}$alkyl, or

$R^4$ and $R^5$ together with the N-atom to which they are attached form a 5- or 6-membered heterocyclic ring which can be substituted by a $C_1$-$C_4$alkyl group,

$R^6$ is hydrogen, $C_1$-$C_{20}$alkyl or $C_3$-$C_{20}$alkenyl, $C_3$-$C_{12}$cycloalkyl, $C_7$-$C_{13}$aralkyl, or $C_6$-$C_{10}$aryl which is unsubstituted or substituted by one or two $C_1$-$C_4$alkyl groups,

$R^7$ is hydrogen or $C_1$-$C_4$alkyl, and

$R^8$ is hydrogen, $C_1$-$C_4$alkyl or $-COR^4$, or

$R^7$ and $R^8$ together with the N-atom to which they are attached from a 5- or 6-membered heterocyclic ring which can be substituted by a $C_1$- $C_4$alkyl group,

$R^9$ is hydrogen or $C_1$-$C_{20}$alkyl and,

$R^{10}$ if x=1, is hydrogen or $C_1$-$C_{20}$alkyl and, if x=0, is $C_1$-$C_5$alkyl, or $R^9$ and $R^{10}$ together form $C_2$-$C_3$alkylene which can be substituted by one or more $C_1$-$C_{20}$alkyl groups, and

$R^{11}$ is $C_1$-$C_{20}$alkyl, $C_6$-$C_{10}$aryl which can be substituted by one or two $C_1$-$C_4$alkyl groups, or is a heterocyclic radical,

or a salt thereof with an acid or a base.

2. A compound of the Formula (I) according to Claim 1, wherein W is hydrogen, halogen, n-alkyl having from 1 to 5 carbon atoms, $-NHCOR^1$ or $-COR^1$, and X and Y are as defined in Claim 1.

3. A compound of the Formula (I) according to Claim 1, wherein $R^2$ is methyl.

4. A compound of the Formula (I) according to Claim 1, wherein W is hydrogen, chlorine, bromine, methyl, ethyl, butyl, $-NHCOR^1$ or phenoxyacetylamino which is unsubstituted or substituted by one or two $C_1$-$C_{12}$ alkyl groups, and $R^1$ is $C_1$-$C_6$alkyl, $C_2$-$C_6$alkenyl, cyclohexyl, benzyl, phenyl or phenyl substituted by one or two methyl or ethyl groups.

5. A compound of the Formula (I) according to Claim 1, wherein W is hydrogen, chlorine, bromine, methyl, $-NHCOCH_2C_6H_5$, $-NHCOC_6H_5$, or phenoxyacetylamino which is unsubstituted or substituted by one or two $C_1$-$C_{12}$ alkyl groups.

6. A compound of the Formula (I) according to Claim 1, wherein n is 1 to 10, k is 1, $R^2$ is methyl, $R^3$ is straight- or branch-alkyl having 1 to 5 carbon atoms, Q is $-COOR^4$ or $-CONR^4R^5$, $-OR^5$, $-OCOR^6$ or $-NR^7R^8$, X is hydrogen and W is hydrogen, chlorine, bromine, methyl, $-NHCOCH_2C_6H_5$ or $-NHCOC_6H_5$, and $R^4$, $R^5$, $R^6$, $R^7$ and $R^8$ are as defined in Claim 1.

7. A compound of the Formula (I) according to Claim 6, wherein n is 3 to 5, k is 1, $R^2$ and $R^3$ are each methyl, X is hydrogen, Q is $-COOR^4$, $-CONR^4R^5$ or $-NR^7R^8$, W is hydrogen, methyl, $-NHCOCH_2C_6H_5$, $-NHCOC_6H_5$, or phenoxyacetylamino which is unsubstituted or substituted by one or two $C_1$-$C_{12}$ alkyl groups, and $R^4$, $R^5$, $R^7$ and $R^8$ are as defined in Claim 1.

8. A process for the production of a compound of the Formula (I), or a salt thereof with an acid or a base, comprising reacting in the presence of an acid or Friedel-Crafts catalyst, in the temperature range from 20 to 150°C, a compound of the Formula (III)

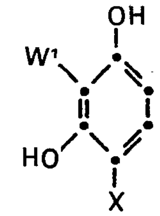

(III)

or a salt thereof with an acid or a base, wherein X is defined as in Claim 1, $W^1$ is hydrogen, n-alkyl having from 1-5 carbon atoms, $-NH_2$, $-NHCOR^1$, $-COR^1$, wherein $R^1$ is defined as in Claim 1, $-NO_2$, or halogen, with a functional alkylating agent capable of introducing a group of the Formula (II), optionally reducing any nitro group $W^1$ to an amino group, and then acylating this amino group to form a group $-NHCOR^1$, and optionally introducing into the 2- or 6-position, substituents W and/or X which are each, respectively, other than hydrogen, and/or optionally converting the compound of Formula (I) so obtained into a corresponding salt with an acid or a base.

9. A process according to Claim 8, wherein the ratio of the aromatic phenol of the Formula (III) to the alkylating species is from 5:1 to 1:1.

10. A process according to Claim 8, wherein one substituent Q in a compound of Formula (I) is converted into a different substituent Q.

## Revendications

1. Composés répondant à la formule (I):

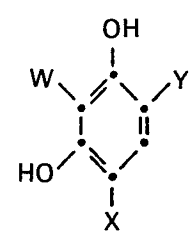

(I)

dans laquelle

W représente l'hydrogène, un halogène, un n-alkyle contenant de 1 à 5 atomes de carbone, un radical $-NHCOR^1$, un radical $-COR^1$ ou un radical phénoxyacétylamino éventuellement porteur d'un ou de deux radicaux alkyles en $C_1$-$C_{12}$,

X représente l'hydrogène, le chlore, le brome, un radical $-SR^{11}$ ou le radical d'un hétérocycle azoté relié par un atome d'azote de son cycle,

Y représente un radical de formule (II):

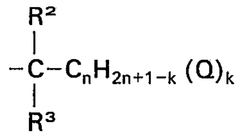

(II)

Q représente un radical $-COOR^4$, $-CONR^4R^5$, $-OR^5$, $-OCOR^6$, $-NR^7R^8$, $-PO(OR^9)[O]_xR^{10}$ (dans lequel x est égal à 0 ou à 1), $-SO_2OH$, $-SO_2NR^4R^5$ ou $-CN$,

n désigne un nombre entier de 1 à 20,

k est égal à 1 ou à 2,

$R^1$ représente un alkyle en $C_1$-$C_{12}$, un alcényle en $C_2$-$C_{12}$, un cycloalkyle en $C_3$-$C_8$, un aralkyle en $C_7$-$C_{13}$ ou un aryle en $C_6$-$C_{10}$ qui peut porter un ou deux radicaux alkyles en $C_1$-$C_4$,

$R^2$ et $R^3$ représentent chacun, indépendamment l'un de l'autre, un alkyle en $C_1$-$C_5$ et, dans le cas où Q représente un radical $-COOR^4$, $R^2$ ou $R^3$ peut porter un ou deux radicaux $-COOR^4$, ou encore au moins l'un des symboles $R^2$ et $R^3$ est fixé au radical $-C_nH_{2n+1-k}$, de telle façon qu'il se forme un radical cycloalkylène en $C_5$-$C_{12}$ porteur de $-(COOR^4)_k$, les radicaux $R^4$ pouvant avoir les mêmes significations ou des significations différentes,

$R^4$ représente l'hydrogène, un alkyle en $C_1$-$C_{20}$ qui peut être interrompu par un ou plusieurs atomes d'oxygène, un alcényle en $C_3$-$C_{20}$, un cycloalkyle en $C_3$-$C_{12}$, un aralkyle en $C_7$-$C_{13}$ ou un aryle en $C_6$-$C_{10}$ éventuellement sustitué, et

$R^5$ représente l'hydrogène ou un alkyle en $C_1$-$C_{20}$, ou

$R^4$ et $R^5$ forment ensemble et avec l'atome d'azote auquel ils sont liés un noyau hétérocyclique pentagonal ou hexanogal qui peut porter un radical alkyle en $C_1$-$C_4$,

$R^6$ représente l'hydrogène, un alkyle en $C_1$-$C_{20}$, un alcényle en $C_3$-$C_{20}$, un cycloalkyle en $C_3$-$C_{12}$, un aralkyle en $C_7$-$C_{13}$ ou un aryle en $C_6$-$C_{10}$, éventuellement porteur d'un ou de deux alkyles en $C_1$-$C_4$,

$R^7$ représente l'hydrogène ou un alkyle en $C_1$-$C_4$, et

$R^8$ représente l'hydrogène, un alkyle en $C_1$-$C_4$ ou un radical $-COR^4$, ou

$R^7$ et $R^8$ forment ensemble et avec l'atome d'azote auquel ils sont liés un noyau hétérocyclique pentagonal ou hexagonal qui peut porter un alkyle en $C_1$-$C_4$,

$R^9$ représente l'hydrogène ou un alkyle en $C_1$-$C_{20}$,

$R^{10}$ représente, lorsque x est égal à 1, l'hydrogène ou un alkyle en $C_1$-$C_{20}$ et, lorsque x est égal à 0, un alkyle en $C_1$-$C_5$, ou

$R^9$ et $R^{10}$ forment ensemble un alkylène en $C_2$ ou $C_3$, qui peut porter un ou plusieurs alkyles en $C_1$-$C_{20}$, et

$R^{11}$ représente un alkyle en $C_1$-$C_{20}$, un aryle en $C_6$-$C_{10}$ éventuellement porteur d'un ou de deux alkyles en $C_1$-$C_4$, ou un radical hétérocyclique, ainsi que les sels de ces composés avec des acides ou des bases.

2. Composés de formule (I) selon la revendication 1, dans lesquels W représente l'hydrogène, un halogène, un alkyle linéaire contenant de 1 à 5 atomes de carbone, un radical $-NHCOR^1$ ou un radical $-COR^1$, tandis que X et Y ont les significations données à la revendication 1.

3. Composés de formule (I) selon la revendication 1 dans lesquels $R^2$ représente un méthyle.

4. Composés de formule (I) selon la revendication 1, dans lesquels W représente l'hydrogène, le chlore, le brome, un méthyle, un éthyle, un butyle, un radical $-NHCOR^1$ ou un radical phénoxyacétylamino éventuellement porteur d'un ou de deux radicaux alkyles en $C_1$-$C_{12}$, et $R^1$ représente un alkyle en $C_1$-$C_6$, un alcényle en $C_2$-$C_6$, un cyclohexyle, un benzyle, un phényle ou un phényle porteur d'un ou de deux radicaux méthyles ou éthyles.

5. Composés de formule (I) selon la revendication 1, dans lesquels W représente l'hydrogène, le chlore, le brome, un méthyle, un radical $-NHCOCH_2C_6H_5$, un radical $-NHCOC_6H_5$ ou un radical phénoxyacétylamino éventuellement porteur d'un ou de deux radicaux alkyles en $C_1$-$C_{12}$.

6. Composés de formule (I) selon la revendication 1, dans lesquels n désigne un nombre entier de 1 à 10, k représente le nombre 1, $R^2$ représente un méthyle, $R^3$ un alkyle en $C_1$-$C_5$, linéaire ou ramifié, Q un radical $-COOR^4$, $-CONR^4R^5$, $-OR^5$, $-OCOR^6$ ou $-NR^7R^8$, X l'hydrogène et W l'hydrogène, le chlore, le brome, un méthyle, un radical $-NHCOCH_2C_6H_5$ ou un radical $-NHCOC_6H_5$, les symboles $R^4$, $R^5$, $R^6$, $R^7$ et $R^8$ ayant les significations données à la revendication 1.

7. Composés de formule (I) selon la revendication 1, dans lesquels n désigne un nombre entier de 3 à 5, k le nombre 1, $R^2$ et $R^3$ représentent chacun un méthyle, X représente l'hydrogène, Q un radical $-COOR^4$, $-CONR^4R^5$ ou $-NR^7R^8$, W l'hydrogène, un méthyle, un radical $-NHCOCH_2C_6H_5$, un radical $-NH-COC_6H_5$ ou un radical phénoxyacétylamino porteur d'un ou de deux alkyles en $C_1$-$C_{12}$, les symboles $R^4$, $R^5$, $R^7$ et $R^8$ ayant les significations données à la revendication 1.

8. Procédé pour préparer des composés de formule (I) et des sels de ces composés avec des acides ou des bases, selon la revendication 1, procédé caractérisé en ce qu'on fait réagir à des températures comprises entre 20 et 150°C, en présence d'un acide ou d'un catalyseur de Friedel-Crafts, un composé répondant à la formule (III)

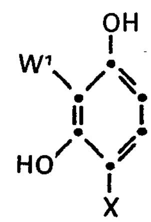

(III)

(ou un sel d'un tel composé avec un acide ou avec une base) dans laquelle X a la signification donnée à la revendication 1, $W^1$ représente l'hydrogène, un alkyle linéaire en $C_1$-$C_5$, un radical $-NH_2$, $-NHCOR^1$, $-COR^1$ ou $-NO^2$ ou un halogène, le

symbole $R^1$ ayant la signification donnée à la revendication 1, avec un agent d'alkylation fonctionnel capable d'introduire des radicaux de formule (II), en ce qu'on réduit des radicaux nitro $W^1$, lorsqu'il y en a, de manière à les convertir en radicaux amino et qu'on transforme ces derniers, par acylation, en radicaux $-NHCOR^1$, qu'on introduit éventuellement, en position 2 et/ou en position 6, des substituants W et/ou X autres que l'hydrogène, et/ou qu'on transforme éventuellement les composés de formule (I) ainsi obtenus en les sels correspondants avec des acides ou des bases.

9. Procédé selon la revendication 8, caractérisé en ce que les composés de formule (III) et l'agent d'alkylation sont mis en jeu dans un rapport compris entre 5:1 et 1:1.

10. Procédé selon la revendication 8, caractérisé en ce qu'on transforme un radical Q en un autre radical Q.